# EUROPÄISCHE PATENTSCHRIFT

(11) **EP 3 329 853 B1**
(45) Veröffentlichungstag und Bekanntmachung des Hinweises auf die Patenterteilung: **30.10.2019**
(21) Anmeldenummer: 17189185.6
(22) Anmeldetag: 04.09.2017
(51) Int. Cl.: A61B 6/04, A61B 6/00, A61B 5/055

(54) **VERFAHREN ZUR AUTOMATISCHEN POSITIONIERUNG EINES PATIENTEN, MEDIZINTECHNISCHE EINRICHTUNG, COMPUTERPROGRAMM UND COMPUTERLESBARES SPEICHERMEDIUM**
METHOD FOR AUTOMATICALLY POSITIONING A PATIENT, MEDICAL DEVICE, COMPUTER PROGRAM AND COMPUTER READABLE STORAGE MEDIUM
PROCÉDÉ DE POSITIONNEMENT AUTOMATIQUE D'UN PATIENT, DISPOSITIF MÉDICAL, PROGRAMME INFORMATIQUE ET MÉMOIRE LISIBLE PAR ORDINATEUR

(43) Veröffentlichungstag der Anmeldung: 06.06.2018
(73) Patentinhaber: Siemens Healthcare GmbH, 91052 Erlangen (DE)
(72) Erfinder: Igler, Harald, 91353 Hausen (DE)

(56) Entgegenhaltungen:
- DE-A1-102006 052 711
- DE-A1-102008 013 613
- DE-A1-102009 032 431
- US-A1- 2013 279 779
- US-A1- 2014 210 468

## Beschreibung

Die Erfindung betrifft ein Verfahren zur automatischen Positionierung eines Patienten relativ zu einer medizintechnischen Einrichtung, eine medizintechnische Einrichtung, ein Computerprogramm und ein computerlesbares Speichermedium.

Für viele Untersuchungen oder Behandlungen eines Patienten ist eine möglichst genaue Positionierung des Patienten erforderlich, um die entsprechende Bildaufnahme oder Behandlung mit der gewünschten Genauigkeit durchführen zu können. Um eine effiziente Positionierung des Patienten zu ermöglichen sowie den Schutz von medizinischem Personal vor elektromagnetischer Strahlung, welche von medizintechnischen Einrichtungen ausgehen kann, zu gewährleisten, ist es wünschenswert, wenn eine solche Positionierung automatisch durchgeführt werden kann und keinen manuellen Eingriff von Bedienpersonal erfordert. Aus dem Stand der Technik sind verschiedene Verfahren zur automatischen Positionierung von Patienten bekannt.

In DE 10 2014 219 666 A1 wird ein Verfahren zur automatischen Patientenpositionierung beschrieben, bei dem ein von einer 3D-Kamera aufgenommenes, Tiefeninformationen umfassendes 3D-Bild eines auf einer Patientenliege gelagerten Patienten verwendet wird. Über auf einem Bildschirm dargestellte Bildinformationen des 3D-Bildes wird ein erster Aufnahmebereich mit einer ersten Startposition sowie mit einer ersten Endposition ausgewählt. Eine erste Position des ersten Aufnahmebereichs relativ zu einer Aufnahmeeinheit wird basierend auf den Tiefeninformationen sowie auf der Auswahl des ersten Aufnahmebereiches bestimmt. Anschließend erfolgt die Positionierung des ersten Aufnahmebereiches in der ersten Position automatisch durch Bewegen der Patientenliege relativ zur Aufnahmeeinheit.

Weitere Verfahren zur automatischen Patientenpositionierung werden in US-A1-2014/210468, in US-A1-2014/0233041 und in DE 10 2006 052711 A1 beschrieben. Diese Dokumente offenbaren die Verwendung von optischen Positionsbestimmungseinrichtungen, die Lichtvorhangsanordnungen umfassen.

Der Erfindung liegt die Aufgabe zugrunde, ein verbessertes Verfahren zur automatischen Positionierung eines Patienten anzugeben.

Erfindungsgemäß ist für ein Verfahren der eingangs genannten Art vorgesehen, dass der Patient in einem Erfassungsbereich einer wenigstens eine dreidimensionale Lichtvorhangsanordnung umfassenden optischen Positionsbestimmungseinrichtung der medizintechnischen Einrichtung auf einer Patientenlagerungseinrichtung der medizintechnischen Einrichtung gelagert ist, wobei eine Startposition des Patienten durch die optische Positionsbestimmungseinrichtung ermittelt wird und eine Positionsabweichung zwischen der ermittelten Startposition und einer in einer Steuerungseinrichtung hinterlegten Zielposition bestimmt wird, wobei in Abhängigkeit der Positionsabweichung eine automatische Positionierung der Patientenlagerungseinrichtung in die Zielposition in bis zu drei Dimensionen erfolgt, wobei eine dreidimensionale Lichtvorhanganordnung verwendet wird, welche mehrere Lichtvorhänge umfasst, deren Erfassungsbereiche sich zumindest teilweise unter einem Winkel schneiden, wobei ein erster Lichtvorhang mit einem im Wesentlichen parallel zu einer zur Patientenlagerung vorgesehenen Fläche der Patientenlagerungseinrichtung angeordnetem Erfassungsbereich sowie ein zweiter Lichtvorhang mit einem unter einem Winkel zu der zur Patientenlagerung vorgesehenen Fläche der Patientenlagerungseinrichtung angeordnetem Erfassungsbereich verwendet werden.

Der Vorteil des erfindungsgemäßen Verfahren besteht darin, dass durch die Lagerung des Patienten auf einer Patientenlagerungseinrichtung, beispielsweise einer Patientenliege, im Erfassungsbereich der wenigstens eine dreidimensionale Lichtvorhangsanordnung umfassenden optischen Positionsbestimmungseinrichtung eine Startposition in drei Dimensionen erfasst werden kann. Dadurch kann beispielsweise ermittelt werden, wie ein Patient beispielsweise auf einer Patientenliege liegt, das heißt wie er auf einer Liegefläche der Patientenliege orientiert und positioniert ist. Die Startposition des Patienten wird durch die optische Positionsbestimmungseinrichtung ermittelt, nachdem der Patient auf der Patientenlagerungseinrichtung Platz genommen hat. Zwischen dieser Startposition und der in einer Steuerungseinrichtung hinterlegten Zielposition wird durch die Steuerungseinrichtung eine Positionsabweichung ermittelt, welche beschreibt, wie groß die Abweichung zwischen der Startposition und der Zielposition jeweils in allen drei Dimensionen ist.

Abhängig von dieser Positionsabweichung erfolgt anschließend eine automatische Positionierung der Patientenlagerungseinrichtung in bis zu drei Dimensionen, beispielsweise in einer Längsrichtung und/oder einer Querrichtung und/oder einer Höhenrichtung. Dies führt dazu, dass eine Patientenposition des auf der Patientenlagerungseinrichtung gelagerten Patienten ausgehend von der Startposition so verändert wird, dass sie mit der Zielposition möglichst genau übereinstimmt. Die Startposition bzw. die Patientenposition kann sich dabei insbesondere auf eine Position eines durch die medizintechnische Einrichtung zu untersuchenden oder zu behandelnden Körperteils des Patienten beziehen, und umfasst, wie erwähnt, üblicherweise auch die Orientierung des Patienten. Ein weiterer Vorteil der Erfindung besteht darin, dass der Patient aufgrund der automatischen Positionierung der Patientenlagerungseinrichtung selbstständig auf der Patientenlagerungseinrichtung Platz nehmen kann, wodurch eine aufwendige manuelle Positionierung des Patienten durch medizinisches Fachpersonal entfällt.

Die automatische Positionierung der Patientenlagerungseinrichtung kann beispielsweise durch Aktoren wie Elektromotoren oder hydraulische Aktoren erfolgen, wobei eine Positionierung der Patientenlagerungseinrichtung in drei Dimensionen wenigstens drei translatorische Freiheitsgrade der Patientenlagerungseinrichtung vorsieht. Zusätzlich können auch drei rotatorische Freiheitsgrade in der Positionierung der Patientenlagerungseinrichtung vorgesehen sein, so dass zusätzlich zu einer translatorischen Bewegung des Patienten auch eine rotatorische Bewegung des Patienten möglich ist. Bei der Steuerungseinrichtung kann es sich um eine externe Steuerungseinrichtung, welche mit der medizintechnischen Einrichtung verbunden ist handeln, oder es kann eine Steuerungseinrichtung verwendet werden, welche Teil der medizintechnischen Einrichtung ist.

Erfindungsgemäß ist vorgesehen, dass eine dreidimensionale Lichtvorhangsanordnung verwendet wird, welche mehrere Lichtvorhänge umfasst, deren Erfassungsbereiche sich zumindest teilweise unter einem Winkel schneiden. Ein Lichtvorhang besteht dabei aus einer Anordnung mehrerer Lichtschranken, welche beispielsweise in wenigstens einer Ebene angeordnet sind. Ein in dieser Ebene platziertes Objekt, welches in einer Richtung orthogonal zur Ausbreitungsrichtung des Lichts der Lichtschranken breiter ist als der Abstand der einzelnen Lichtschranken zueinander, kann durch den Lichtvorhang erfasst werden, wenn es in dessen Erfassungsbereich positioniert wird. Das im Erfassungsbereich des Lichtvorhangs platzierte Objekt unterbricht eine oder mehrere Lichtschranken, wodurch seine Position in der wenigstens einen Ebene des Lichtvorhangs bestimmt werden kann. Bei einer dreidimensionalen Lichtvorhangsanordnung, welche mehrere Lichtvorhänge umfasst, deren Erfassungsbereiche sich zumindest teilweise unter einem Winkel schneiden, kann in entsprechender Weise die Position eines Objektes in drei Dimensionen bestimmt werden.

Es kann dabei als Teil der Lichtvorhangsanordnung insbesondere auch wenigstens ein Lichtvorhang zum Einsatz kommen, welcher einen durch in mehreren, beispielsweise parallelen Ebenen angeordnete Lichtschranken gebildeten, zweidimensionalen Erfassungsbereich aufweist. In einem bevorzugten Fall ist es für eine dreidimensionale Positionsbestimmung ausreichend, wenn zwei einen solchen zweidimensionalen Erfassungsbereich aufweisende Lichtvorhänge derart angeordnet werden, dass sich ihre Erfassungsbereiche zumindest teilweise unter einem Winkel schneiden, so dass ein insgesamt dreidimensionaler Erfassungsbereich der Lichtvorhangsanordnung im Überlappungsbereich der zweidimensionalen Erfassungsbereiche der Lichtvorhänge entsteht.

Erfindungsgemäß ist vorgesehen, dass ein erster Lichtvorhang mit einem im Wesentlichen parallel zu einer zur Patientenlagerung vorgesehenen Fläche der Patientenlagerungseinrichtung angeordnetem Erfassungsbereich sowie ein zweiter Lichtvorhang mit einem unter einem Winkel zu der zur Patientenlagerung vorgesehenen Fläche der Patientenlagerungseinrichtung angeordnetem Erfassungsbereich verwendet werden. Der erste Lichtvorhang und/oder der zweite Lichtvorhang weisen dabei beispielsweise jeweils einen zweidimensionalen Erfassungsbereich auf, so dass die Position des auf der Patientenlagerungseinrichtung gelagerten Patienten in drei Dimensionen bestimmt werden kann. Die zur Patientenlagerung vorgesehene Fläche der Patientenlagerungseinrichtung kann beispielsweise die Liegefläche einer Patientenliege oder die Sitzfläche eines Patientenstuhls sein. Abhängig von der Ausgestaltung der Patientenlagerungseinrichtung können dabei ein erster Lichtvorhang und ein zweiter Lichtvorhang mit verschieden großen beziehungsweise verschieden geformten Erfassungsbereichen verwendet werden.

In einer weiteren bevorzugten Ausgestaltung der Erfindung kann vorgesehen sein, dass der zweite Lichtvorhang eine Patientenaufnahme der medizintechnischen Einrichtung zumindest teilweise durchstrahlt. Die Patientenaufnahme kann dabei eine Öffnung der medizintechnischen Einrichtung, beispielsweise eine Bohrung eines Magnetresonanztomographen oder eine Gantry eines Computertomographen, sein, in welche der Patient beziehungsweise die Patientenlagerungseinrichtung, auf der der Patient gelagert ist, hineinbewegt werden kann. Der zweite Lichtvorhang wird dabei derart angeordnet, dass er die Patientenaufnahme zumindest teilweise durchstrahlt. Dies ermöglicht es, durch den Lichtvorhang die Position des Patienten auch innerhalb der Patientenaufnahme zu bestimmen. Der Erfassungsbereich des zweiten Lichtvorhangs liegt auf diese Weise zumindest teilweise im Inneren der Patientenaufnahme. Es ist möglich, dass der zweite Lichtvorhang derart angeordnet wird, dass der Erfassungsbereich zusätzlich zu einem Teil des Inneren der Patientenaufnahme auch einen Bereich außerhalb, insbesondere vor der Patientenaufnahme erfasst, so dass eine Startposition eines noch nicht in die Patientenaufnahme hineinbewegten Patienten, beispielsweise bei Anordnung des Patienten auf einer Patientenlagerungseinrichtung, welche sich vor der Patientenaufnahme befindet, durch den zweiten Lichtvorhang erfasst werden kann.

Erfindungsgemäß kann vorgesehen sein, dass während einer von der medizintechnischen Einrichtung durchzuführenden Bildaufnahme und/oder Behandlung nach der ersten automatischen Positionierung der Patientenlagerungseinrichtung fortlaufend eine Momentanposition des Patienten durch die optische Positionsbestimmungseinrichtung ermittelt wird. Dies bedeutet, dass auch für einen bereits zur Zielposition bewegten Patienten fortlaufend eine eine momentane Patientenposition beschreibende Momentanposition des Patienten durch die optische Positionsbestimmungseinrichtung ermittelt wird. Dies setzt voraus, dass sich die Zielposition des Patienten im Erfassungsbereich der optischen Positionsbestimmungseinrichtung befindet. Wenn während einer von der medizintechnischen Einrichtung durchzuführenden Bildaufnahme und/oder Behandlung eine Abweichung zwischen der Momentanposition des Patienten und der Zielposition festgestellt wird, kann dies beispielsweise bei Überschreiten eines ersten Grenzwertes der Positionsabweichung eine akustische oder optische Hinweisgabe an den Patienten und/oder eine Bedienperson zur Folge haben, welche ihn dazu auffordert, wieder die Zielposition einzunehmen. Es ist auch möglich, dass eine Positionsabweichung zwischen der Momentanposition des Patienten und der Zielposition über dem ersten Grenzwert oder über einem zweiten Grenzwert einen Abbruch der Bildaufnahme und/oder der Behandlung zur Folge hat. Eine Hinweisgabe, die bevorzugt eine konkrete Eigenpositionierungsanweisung an den Patienten umfassen kann, kann bevorzugt in Situationen erfolgen, in denen die Patientenposition nicht durch eine automatische Positionierung der Patientenlagerungseinrichtung in die Zielposition bewegt werden kann, beispielsweise weil die Positionsabweichung zu groß ist, um durch eine Positionierung der Patientenlagerungseinrichtung ausgeglichen zu werden.

In vorteilhafter Weise kann auch vorgesehen sein, dass bei einer während der Bildaufnahme und/oder Behandlung auftretenden Positionsabweichung zwischen der Momentanposition des Patienten und der wenigstens einen in der Steuerungseinrichtung hinterlegten Zielposition eine erneute automatische Positionierung der Patientenlagerungseinrichtung erfolgt. Dies ermöglicht vorteilhaft eine automatische Nachführung des Patienten während der Bildaufnahme und/oder der Behandlung und ermöglicht den Ausgleich von Positionsabweichungen zwischen der Patientenposition und der Zielposition, welche beispielsweise durch Bewegungen des Patienten hervorgerufen werden können.

Erfindungsgemäß kann vorgesehen sein, dass die Zielposition durch die Steuereinrichtung in Abhängigkeit einer eine von der medizintechnischen Einrichtung durchzuführende Bildaufnahme und/oder Behandlung beschreibenden Vorgangsinformation ermittelt und in der Steuereinrichtung hinterlegt wird. Die Zielposition kann somit in Abhängigkeit von notwendigen Parametern, wie beispielsweise der Position eines Isozentrums der durchzuführenden Bildaufnahme und/oder Behandlung ermittelt werden. Es können folglich für verschiedene Bildaufnahmen und/oder Behandlungen unterschiedliche Zielpositionen ermittelt und in der Steuereinrichtung hinterlegt werden. Dadurch wird die Positionierung des Patienten in Abhängigkeit der durchzuführenden Bildaufnahme und oder Behandlung ermöglicht, wobei die Art der durchzuführenden Bildaufnahme und/oder Behandlung der Steuereinrichtung beispielsweise durch Bedienpersonal über eine Benutzerschnittstelle oder eine Kommunikationsverbindung mitgeteilt werden kann.

In einer bevorzugten Ausgestaltung der Erfindung kann vorgesehen sein, dass mehrere Zielpositionen in der Steuereinrichtung hinterlegt sind, welche während einer von der medizintechnischen Einrichtung durchzuführenden Bildaufnahme und/oder Behandlung zeitversetzt für eine mehrere automatische Positionierungen der Patientenlagerungseinrichtung umfassende Positionierungsfolge verwendet werden. Dadurch wird es ermöglicht, dass während einer Bildaufnahme und/oder Behandlung mehrere Zielpositionen zeitversetzt durch eine automatische Positionierung der Patientenlagerungseinrichtung angefahren werden können, so dass Bildaufnahmen von unterschiedlichen Bereichen des Patienten und/oder in unterschiedlichen Tiefen oder Ähnlichem vorgenommen werden können. Zum Anfahren einer jeweiligen Zielposition kann dabei sowohl die Startposition unter Berücksichtigung aller bereits erfolgten Positionierungen herangezogen werden oder aber, bei einer gegebenenfalls ermittelten Momentanposition des Patienten, auf diese zurückgegriffen werden. Die Positionsfolge kann aber auch einen Bewegungsweg des Patienten beschreiben, wenn während der Bildaufnahme und/oder Behandlung eine gezielte Bewegung stattfinden soll.

Erfindungsgemäß kann die Verwendung wenigstens eines der medizintechnischen Einrichtung zugeordneten Zusatzgerätes, insbesondere eines Messaufnehmers, vorgesehen sein, wobei über ein Positionsbestimmungsmittel eine Bestimmung einer Zusatzgeräteposition erfolgt und die Zielposition durch die Steuereinrichtung in Abhängigkeit von der Zusatzgeräteposition ermittelt wird. Dies ermöglicht es, die Position eines Zusatzgerätes, wie eines Messaufnehmers, über ein Positionsbestimmungsmittel zu ermitteln und die Zielposition in Abhängigkeit der Zusatzgeräteposition zu ermitteln. Die automatische Positionierung des Patienten erfolgt somit unter Berücksichtigung der Zusatzgeräteposition wenigstens eines Zusatzgerätes, wobei für verschiedene Zusatzgeräte unterschiedliche Arten der Abhängigkeiten der Zielposition vorgesehen sein können. Das Positionsbestimmungsmittel kann dabei Teil der medizintechnischen Einrichtung oder Teil des der medizintechnischen Einrichtung zugeordneten Zusatzgerätes sein, wobei im letzten Fall eine Übermittlung der Zusatzgeräteposition an die medizintechnische Einrichtung und/oder die Steuereinrichtung vorgesehen sein kann. Beispielsweise kann als ein Positionsbestimmungsmittel des Zusatzgerätes eine Vorrichtung verwendet werden, welche zur Durchführung einer triangularen Zeitstreckenmessung, beispielsweise durch Auswertung von Phasenunterschieden gepulster Lichtsignale, sowie zur Übermittlung der bestimmten Zusatzgeräteposition an die medizintechnische Einrichtung und/oder die Steuereinrichtung ausgebildet ist.

In einer bevorzugten Ausgestaltung der Erfindung kann vorgesehen sein, dass als Positionsbestimmungsmittel ein optisches Positionsbestimmungsmittel des Zusatzgerätes, insbesondere wenigstens ein Lichtsensor und/oder ein Lichtempfänger verwendet wird, wobei die Zusatzgeräteposition durch die optische Positionsbestimmungseinrichtung der medizintechnischen Einrichtung bestimmt wird. Es kann somit vorteilhaft die als dreidimensionale Lichtvorhangsanordnung ausgebildete optische Positionsbestimmungseinrichtung der medizintechnischen Einrichtung verwendet werden, um die Position des ein optisches Positionsbestimmungsmittel aufweisenden Zusatzgerätes zu ermitteln. Dabei kann das Zusatzgerät beispielsweise einen Lichtsender und/oder einen Lichtempfänger aufweisen, wobei vom Zusatzgerät gesendetes Licht von der dreidimensionalen Lichtvorhangsanordnung erfasst werden kann beziehungsweise wobei von der dreidimensionalen Lichtvorhangsanordnung gesendetes Licht vom Zusatzgerät empfangen werden kann. Dies ermöglicht es in einfacher Weise, zusätzlich zu der Patientenposition auch die Zusatzgeräteposition zu ermitteln.

Weiterhin kann erfindungsgemäß vorgesehen sein, dass während der von der medizintechnischen Einrichtung durchzuführenden Bildaufnahme und/oder Behandlung wenigstens eine aktualisierte Zielposition in Abhängigkeit von der Zusatzgeräteposition des wenigstens einen Zusatzgerätes ermittelt und in der Steuereinrichtung hinterlegt wird, wobei bei einer Positionsabweichung zwischen der Momentanposition des Patienten und der wenigstens einen aktualisierten Zielposition eine erneute automatische Positionierung der Patientenlagerungseinrichtung erfolgt. Bei einer Positionsänderung des Zusatzgerätes, beispielsweise bei einem Verrutschen eines auf dem Patienten angeordneten Zusatzgerätes aufgrund einer Patientenbewegung, kann eine aktualisierte Zielposition in Abhängigkeit der geänderten Zusatzgeräteposition erfolgen. Wenn die ermittelte aktualisierte Zielposition von der Momentanposition des Patienten abweicht, kann eine Änderung der Momentanposition des Patienten durch eine erneute automatische Positionierung der Patientenlagerungseinrichtung erfolgen. Es wird dadurch eine Nachführung des Patienten in Abhängigkeit der Zusatzgeräteposition ermöglicht. Es kann auch vorgesehen sein, dass zusätzlich oder alternativ eine Ermittlung einer Positionsabweichung zwischen der Zusatzgeräteposition und der Momentanposition des Patienten erfolgt, wobei bei einer Positionsabweichung, welche einen vorgegebenen Grenzwert überschreitet, ab welchem eine Fortführung der Bildaufnahme und/oder Behandlung nicht sinnvoll ist, ein optischer oder akustischer Hinweis an den Patienten gegeben werden kann, welcher ihn dazu veranlassen soll, seine Position und/oder die Position des Zusatzgerätes derart zu verändern, dass die Bildaufnahme und/oder die Behandlung sinnvoll fortgeführt werden kann. Es ist möglich, dass für ein eigener, dem Zusatzgerät zugeordneter Grenzwert verwendet wird, oder dass einer oder mehrere Grenzwerte für das Zusatzgerät verwendet werden, welche dem ersten Grenzwert und/oder dem zweiten Grenzwert der Abweichung der Momentanposition des Patienten zu der Zielposition entsprechen können. Eine Hinweisgabe, die bevorzugt eine konkrete Eigenpositionierungsanweisung an den Patienten umfassen kann, kann bevorzugt in Situationen erfolgen, in denen die Patientenposition nicht durch eine automatische Positionierung der Patientenlagerungseinrichtung in die Zielposition bewegt werden kann, beispielsweise weil die Positionsabweichung zu groß ist, um durch eine Positionierung der Patientenlagerungseinrichtung ausgeglichen zu werden. Zusätzlich oder alternativ dazu ist es auch möglich, dass in diesem Fall ein Abbruch der Bildaufnahme und/oder der Behandlung erfolgt. Bevorzugt ist es jedoch, wenn aufgrund der Bewegungsmöglichkeiten der Patientenlagerungseinrichtung die Zielposition korrekt eingenommen werden kann, den Patienten automatisch wieder korrekt zu positionieren.

Erfindungsgemäß kann vorgesehen sein, dass bei jeder automatischen Positionierung der Patientenlagerungseinrichtung eine diese Positionierung und/oder die Momentanposition des Patienten und/oder die Zielposition beschreibende Positionierungsinformation in der Steuereinrichtung gespeichert wird. Somit wird es folglich durch die Positionierungsinformation oder die Positionierungsinformationen ermöglicht, zur Dokumentation und/oder zur Nachvollziehbarkeit der Messung die Positionierung der Patientenlagerungseinrichtung heranzuziehen. Die Positionierungsinformation kann dabei zusätzlich die der automatischen Positionierung zugrundeliegende Momentanposition des Patienten und/oder die der automatischen Positionierung zugrundeliegende Zielposition beschreiben, so dass eine genaue Dokumentation der automatischen Positionierung des Patienten während der Bildaufnahme und/oder der Behandlung ermöglicht wird. Es ist auch möglich, dass die Positionierungsinformation Zusatzdaten zu der durchgeführten Bildaufnahme und/oder der durchgeführten Behandlung und/oder zu Betriebsparametern der medizintechnischen Einrichtung enthält, so dass der gesamte Vorgang nachvollzogen und zur Auswertung einer Bildaufnahme und/oder Behandlung herangezogen werden kann.

Für eine erfindungsgemäße medizintechnische Einrichtung ist vorgesehen, dass sie zur Durchführung eines erfindungsgemäßen Verfahrens eingerichtet ist. Sämtliche Ausführung zum Verfahren gelten entsprechen fort.

Für ein erfindungsgemäßes Computerprogramm ist vorgesehen, dass es Befehle umfasst, welche bei Ausführung des Programms durch eine Recheneinrichtung diese veranlassen, ein erfindungsgemäßes Verfahren auszuführen.

Für ein erfindungsgemäßes computerlesbares Speichermedium ist vorgesehen, dass es ein erfindungsgemäßes Computerprogramm enthält.

Weitere Vorteile und Einzelheiten ergeben sich aus den im Folgenden beschriebenen Ausführungsbeispielen sowie anhand der Zeichnungen. Dabei zeigen:
- Fig. 1: eine perspektivische Prinzipdarstellung einer erfindungsgemäßen medizintechnischen Einrichtung,
- Fig. 2: eine Prinzipdarstellung einer Seitenansicht einer erfindungsgemäßen medizintechnischen Einrichtung,
- Fig. 3: eine Prinzipdarstellung einer Aufsicht auf ein erstes Ausführungsbeispiel einer erfindungsgemäßen medizintechnischen Einrichtung,
- Fig. 4: eine Prinzipdarstellung einer Aufsicht auf ein zweites Ausführungsbeispiel einer erfindungsgemäßen medizintechnischen Einrichtung, sowie
- Fig. 5: ein Ablaufdiagramm eines erfindungsgemäßen Verfahrens.

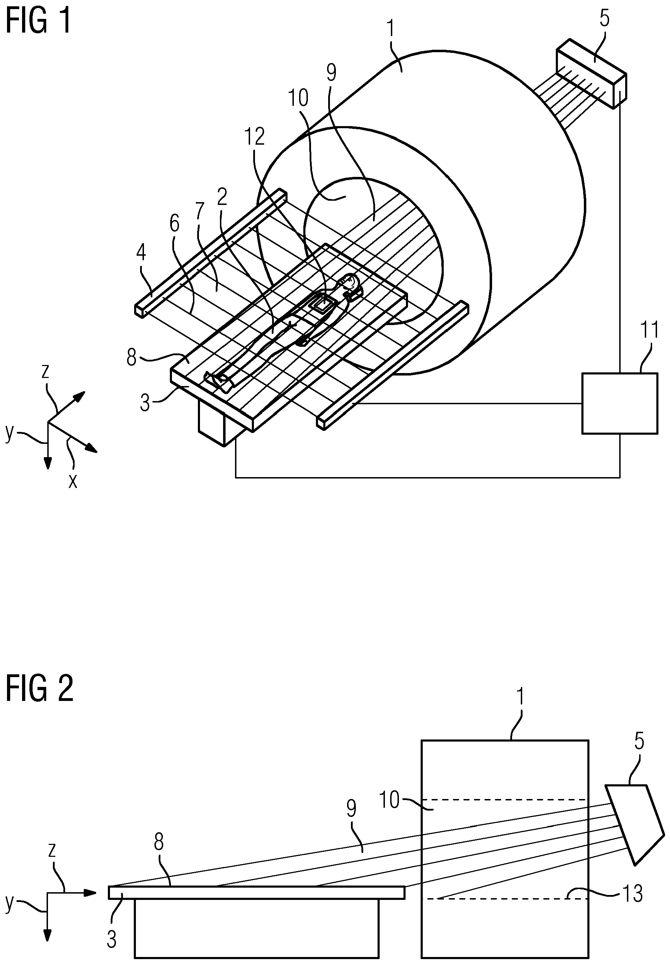

Fig. 1 zeigt eine perspektivische Prinzipdarstellung einer erfindungsgemäßen medizintechnischen Einrichtung 1. Diese umfasst zur automatischen Positionierung eines Patienten 2 eine als Patientenliege 3 ausgebildete Patientenlagerungsvorrichtung, welche in drei Dimensionen durch Aktoren, wie beispielsweise Elektromotoren und/oder hydraulische Aktoren (nicht gezeigt), bewegt werden kann. Die medizintechnische Einrichtung 1 umfasst weiterhin eine dreidimensionale Lichtvorhangsanordnung als optische Positionsbestimmungseinrichtung, wobei die dreidimensionale Lichtvorhangsanordnung einen ersten Lichtvorhang 4 sowie einen zweiten Lichtvorhang 5 umfasst.

Der erste Lichtvorhang 4 weist mehrere Lichtschranken 6 auf, durch welche ein Erfassungsbereich 7 gebildet wird, welcher im Wesentlichen parallel zu einer Oberfläche 8 der Patientenliege 3 verläuft. Der Übersichtlichkeit halber ist nur eine Ebene des Erfassungsbereiches 7 dargestellt, es kann insbesondere vorgesehen sein, dass der erste Lichtvorhang 4 weitere Lichtschranken 6 umfasst, welche derart angeordnet sind, dass der Erfassungsbereich 7 durch mehrere parallel zueinander angeordnete Ebenen in y-Richtung erweitert ist.

Der Erfassungsbereich 9 des zweiten Lichtvorhangs 5 durchstrahlt teilweise eine Patientenaufnahme 10 der medizintechnischen Einrichtung 1 und ist unter einem Winkel zur Oberfläche 8 der Patientenliege 3 angeordnet. Um eine Detektion des vom zweiten Lichtvorhang 5 ausgesendeten Lichts zu ermöglichen, kann vorgesehen sein, dass die Oberfläche 8 der Patientenliege 3 reflektierend ist, so dass das vom zweiten Lichtvorhang 5 ausgesendete Licht an den Lichtvorhang 5 zurückgesendet wird. Zusätzlich oder alternativ dazu kann vorgesehen sein, dass die Oberfläche 8 der Patientenliege 3 Lichtsensoren zur Detektion des auf die Oberfläche 8 treffenden Lichtes des zweiten Lichtvorhangs 5 aufweist. Auch für den Lichtvorhang 5 kann vorgesehen sein, dass der Erfassungsbereich 9 mehrere Ebenen umfasst, beispielsweise derart, dass die gesamte Oberfläche 8 der Patientenliege 3 vom Erfassungsbereich 9 des zweiten Lichtvorhangs 9 erfasst wird.

Die durch den ersten Lichtvorhang 4 und den zweiten Lichtvorhang 5 gebildete dreidimensionale Lichtvorhangsanordnung ermöglicht eine dreidimensionale Bestimmung einer Patientenposition des Patienten 2.

Eine Startposition des Patienten 2, wie sie beispielsweise in Fig. 1 dargestellt ist, kann durch die dreidimensionale Lichtvorhangsanordnung bestimmt und an eine Steuereinrichtung 11 übermittelt werden. Die Steuereinrichtung 11 kann ein Teil der medizintechnischen Einrichtung 1 sein, oder es kann sich um eine externe und mit der medizintechnischen Einrichtung 1 verbundene Steuereinrichtung, beispielsweise einen Computer, handeln. Die Steuereinrichtung 11 kann eine Positionsabweichung zwischen der Startposition des Patienten und einer in der Steuereinrichtung 11 hinterlegten Zielposition ermitteln. Abhängig von dieser Positionsabweichung kann anschließend eine automatische Positionierung der Patientenlagerungseinrichtung in drei Dimensionen durch Ansteuerung der Aktoren der Patientenliege erfolgen, wodurch eine Positionierung des Patienten 2 an der Zielposition ermöglicht wird.

Die automatische Positionierung der Patientenliege und somit des Patienten 2 kann dabei beispielsweise ein Bewegen der Patientenliege 3 in z-Richtung, also in die Aufnahme 10 der medizintechnischen Einrichtung 1 hinein, und/oder eine Positionierung der Patientenliege 3 in x-Richtung und/oder in y-Richtung beinhalten. Als Startposition des Patienten kann eine Patientenposition, wie beispielsweise die Position eines Körperteils oder eines Bereichs des Patienten, herangezogen werden, welche das Ziel einer von der medizintechnischen Einrichtung 1 durchzuführenden Bildaufnahme und/oder Behandlung ist. Die in der Steuereinrichtung 11 hinterlegte Zielposition kann in Abhängigkeit der von der medizintechnischen Einrichtung 1 durchzuführenden Bildaufnahme und/oder Behandlung bestimmt werden, so dass beispielsweise ein Isozentrum der medizintechnischen Einrichtung 1 in Bezug auf die durchzuführende Bildaufnahme und/oder Behandlung als Zielposition verwendet werden kann.

Der medizintechnischen Einrichtung 1 kann ein Zusatzgerät 12 zugeordnet sein, dessen Zusatzgeräteposition durch ein Positionsbestimmungsmittel bestimmbar ist. In der in Fig. 1 dargestellten Ausführung können als Positionsbestimmungsmittel beispielsweise einer oder mehrerer Lichtempfänger und/oder Lichtsensoren (nicht dargestellt) des Zusatzgerätes 12 vorhanden sein, welche es ermöglichen, die Position des Zusatzgerätes durch die dreidimensionale Lichtvorhangsanordnung der medizintechnischen Einrichtung 1 zu bestimmen.

Die in der Steuereinrichtung 11 hinterlegte Zielposition kann in Abhängigkeit der Zusatzgerätepositionen des Zusatzgerätes 12 bestimmt werden.

In Fig. 2 ist eine schematische Seitenansicht eines Ausführungsbeispiels einer erfindungsgemäßen medizintechnischen Einrichtung 1 dargestellt. In dieser Darstellung ist ersichtlich, dass der zweite Lichtvorhang 5 auch in y-Richtung beabstandet angeordnete Lichtschranken umfasst. Der erste Lichtvorhang 4 ist aus Gründen der Übersichtlichkeit nicht dargestellt. Der Erfassungsbereich 9 des Lichtvorhangs 5 ist derart ausgedehnt, dass er sowohl das Innere der Patientenaufnahme 10 zumindest teilweise abdeckt als auch den Bereich vor der Patientenaufnahme 10, in dem die Patientenliege 3 bei der Beladung mit dem Patienten angeordnet ist, umfasst. Zur Rücksendung des vom zweiten Lichtvorhang 5 ausgesendeten Lichtes kann hierbei insbesondere vorgesehen sein, dass die Oberfläche 8 der Patientenliege 3 Lichtsensoren aufweist und/oder zumindest teilweise reflektierende Bereiche aufweist. Es ist zusätzlich oder alternativ möglich, dass ein dritter Lichtvorhang als Teil der Lichtvorhangsanordnung verwendet wird, welcher an der dem zweiten Lichtvorhang 5 gegenüberliegenden Seite der medizintechnischen Einrichtung 1 angeordnet ist und dessen Erfassungsbereich sich ebenfalls zumindest teilweise in das Innere der Patientenaufnahme 10 hinein erstreckt.

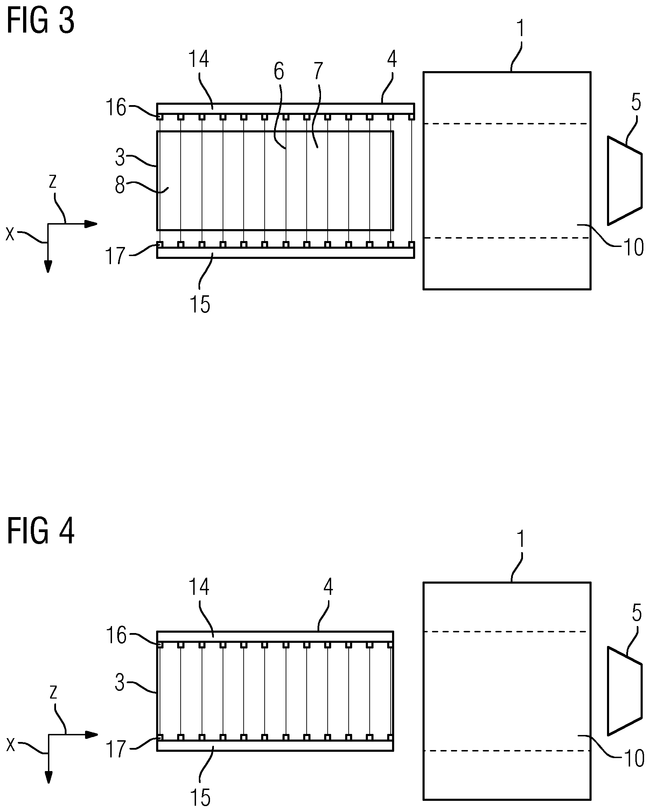

Fig. 3 zeigt eine schematische Aufsicht auf ein Ausführungsbeispiel einer erfindungsgemäßen medizintechnischen Einrichtung 1. Der Erfassungsbereich des zweiten Lichtvorhangs 5 ist aus Gründen der Übersichtlichkeit nicht dargestellt. Der erste Lichtvorhang 4 umfasst einen außerhalb der Patientenaufnahme angeordneten Sendeteil 14 sowie einen außerhalb der Patientenaufnahme 10 angeordneten Reflexionsteil 15. Zur Bildung der den Erfassungsbereich 7 abdeckenden Lichtschranken 6 weist der Sendeteil 14 mehrere Lichtsender 16 auf sowie der Reflexionsteil 15 mehrere dem Lichtsender 16 gegenüberliegende Lichtreflektoren 17. Anstatt Lichtreflektoren 17 können auch Lichtsensoren eingesetzt werden. Der Erfassungsbereich 7 des ersten Lichtvorhangs 4 ist im Wesentlichen parallel zu einer Oberfläche 8 der Patientenliege 3 angeordnet. Die Lichtsender 16 sowie die Lichtreflektoren 17 können dabei insbesondere so angeordnet werden, dass sich der Erfassungsbereich 7 des ersten Lichtvorhangs 4 auch in y-Richtung erstreckt, mithin also eine Positionsbestimmung eines auf der Patientenliege 3 gelagerten Patienten 2 in y-Richtung und in z-Richtung ermöglicht. Eine Erfassung der Patientenposition in x-Richtung sowie in y-Richtung wird, wie beispielsweise in den Fig. 1 und 2 dargestellt, mithilfe des zweiten Lichtvorhangs 5 ermöglicht. Es ist alternativ möglich, dass der erste Lichtvorhang 4, bzw. der Sendeteil 14 und der Reflexionsteil 15, derart ausgebildet sind, dass sich der Erfassungsbereich 7 des ersten Lichtvorhangs 4 auch innerhalb der Patientenaufnahme 10 erstreckt.

Fig. 4 zeigt eine schematische Aufsicht auf eine alternative Ausführung einer erfindungsgemäßen medizintechnischen Einrichtung 1. Auch in dieser Abbildung ist der Erfassungsbereich 9 des zweiten Lichtvorhangs 5 der Übersichtlichkeit halber nicht dargestellt. In dieser alternativen Ausführung sind der Sendeteil 14 sowie der Reflexionsteil 15 auf der Patientenliege 3 angeordnet. Dies ermöglicht eine Positionsbestimmung des Patienten auch bei einer Positionierung der Patientenliege 3 innerhalb der Patientenaufnahme 10.

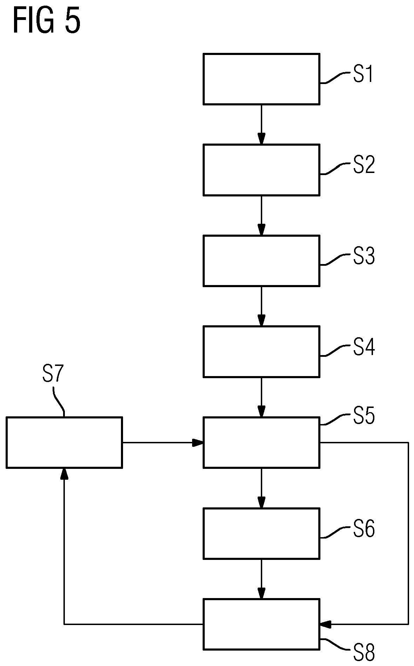

In Fig. 5 ist ein Ablaufdiagramm eines erfindungsgemäßen Verfahrens dargestellt. Schritt S1 stellt den Start des erfindungsgemäßen Verfahrens dar. In diesem Schritt wird die Startposition des auf der Patientenliege 3 gelagerten Patienten 2 durch die dreidimensionale Lichtvorhangsanordnung ermittelt. Anschließend erfolgt in Schritt S2 durch die Steuereinrichtung 11 die Ermittlung einer Positionsabweichung zwischen der Startposition des Patienten und einer in der Steuereinrichtung 11 hinterlegten Zielposition. Die Zielposition kann dabei in Abhängigkeit der von der medizintechnischen Einrichtung 1 durchzuführenden Bildaufnahme und/oder Behandlung sowie in Abhängigkeit der Position eines Zusatzgerätes 12 ermittelt und in der Steuereinrichtung 11 hinterlegt worden sein. Nach Ermittlung der Positionsabweichung zwischen Startposition und Zielposition erfolgt in Schritt S3 die automatische Positionierung der Patientenliege 3, so dass die Patientenposition mit der Zielposition übereinstimmt. Im darauffolgenden Schritt S4 erfolgt der Start der von der medizintechnischen Einrichtung 1 durchzuführenden Bildaufnahme und/oder Behandlung.

Während der Bildaufnahme und/oder Behandlung erfolgt die Bestimmung einer Momentanposition des Patienten und/oder einer Zusatzgeräteposition des Zusatzgerätes 12 in Schritt S5. Ausgehend von der ermittelten Momentanposition des Patienten wird in Schritt S6 eine Positionsabweichung zwischen der Momentanposition des Patienten und der Zielposition ermittelt. Wenn die ermittelte Positionsabweichung einen vorgegebenen Grenzwert überschreitet, erfolgt in Schritt S7 eine erneute automatische Positionierung der Patientenliege 3. Zusätzlich oder alternativ dazu kann in Schritt 6 aus einer ermittelten Zusatzgeräteposition eine aktualisierte Zielposition ermittelt werden, welche zur Bestimmung einer Positionsabweichung zwischen Momentanposition des Patienten und der aktualisierten Zielposition herangezogen wird. Nach erfolgter automatischer Positionierung der Patientenlagerungseinrichtung beziehungsweise des Patienten in Schritt S7 erfolgt eine erneute Ermittlung der Momentanposition des Patienten und/oder der Zusatzgeräteposition eines Zusatzgerätes in Schritt S5 für die Dauer der durchzuführenden Bildaufnahme und/oder Behandlung.

Bei Beendigung der Bildaufnahmen und/oder der Behandlung durch die medizintechnische Einrichtung 1 erfolgt das Ende des Verfahrens in Schritt S8. Es kann vorgesehen sein, dass in Schritt S3 sowie in Schritt S7 eine die jeweilige Positionierung und/oder die Momentanposition des Patienten und/oder die Zielposition beschreibende Positionierungsinformation in der Steuereinrichtung 11 gespeichert wird. Dies dient der Dokumentation der durchgeführten Bildaufnahme und/oder Behandlung, um insbesondere zur Auswertung der durchgeführten Bildaufnahme und/oder der Behandlung die Positionierungen sowie die Momentanposition des Patienten und/oder die Zielposition nachzuvollziehen.

Obwohl die Erfindung im Detail durch das bevorzugte Ausführungsbeispiel näher illustriert und beschrieben wurde, so ist die Erfindung nicht durch die offenbarten Beispiele eingeschränkt und andere Variationen können vom Fachmann hieraus abgeleitet werden, ohne den Schutzumfang der Erfindung zu verlassen.

## Patentansprüche

1. Verfahren zur automatischen Positionierung eines Patienten (2) relativ zu einer medizintechnischen Einrichtung (1), wobei der Patient (2) in einem Erfassungsbereich einer wenigstens eine dreidimensionale Lichtvorhangsanordnung umfassenden optischen Positionsbestimmungseinrichtung der medizintechnischen Einrichtung (1) auf einer Patientenlagerungseinrichtung der medizintechnischen Einrichtung (1) gelagert ist, wobei eine Startposition des Patienten (2) durch die optische Positionsbestimmungseinrichtung ermittelt wird und eine Positionsabweichung zwischen der ermittelten Startposition und einer in einer Steuerungseinrichtung (11) hinterlegten Zielposition bestimmt wird, wobei in Abhängigkeit der Positionsabweichung eine automatische Positionierung der Patientenlagerungseinrichtung in die Zielposition in bis zu drei Dimensionen erfolgt, **dadurch gekennzeichnet, dass** eine dreidimensionale Lichtvorhanganordnung verwendet wird, welche mehrere Lichtvorhänge (4, 5) umfasst, deren Erfassungsbereiche sich zumindest teilweise unter einem Winkel schneiden, wobei ein erster Lichtvorhang (4) mit einem im Wesentlichen parallel zu einer zur Patientenlagerung vorgesehenen Fläche der Patientenlagerungseinrichtung angeordnetem Erfassungsbereich (7) sowie ein zweiter Lichtvorhang (5) mit einem unter einem Winkel zu der zur Patientenlagerung vorgesehenen Fläche der Patientenlagerungseinrichtung angeordnetem Erfassungsbereich (9) verwendet werden.

2. Verfahren nach Anspruch 1, **dadurch gekennzeichnet, dass** der zweite Lichtvorhang (5) eine Patientenaufnahme (10) der medizintechnischen Einrichtung (1) zumindest teilweise durchstrahlt.

3. Verfahren nach Anspruch 1 oder 2, **dadurch gekennzeichnet, dass** während einer von der medizintechnischen Einrichtung (1) durchzuführenden Bildaufnahme und/oder Behandlung nach der ersten automatischen Positionierung der Patientenlagerungseinrichtung fortlaufend eine Momentanposition des Patienten (2) durch die optische Positionsbestimmungseinrichtung ermittelt wird.

4. Verfahren nach Anspruch 3, **dadurch gekennzeichnet, dass** bei einer während der Bildaufnahme und/oder Behandlung auftretenden Positionsabweichung zwischen der Momentanposition des Patienten (2) und der wenigstens einen in der Steuerungseinrichtung (11) hinterlegten Zielposition eine erneute automatische Positionierung der Patientenlagerungseinrichtung erfolgt.

5. Verfahren nach einem der vorangehenden Ansprüche, **dadurch gekennzeichnet, dass** die Zielposition durch die Steuereinrichtung (11) in Abhängigkeit einer eine von der medizintechnischen Einrichtung (1) durchzuführenden Bildaufnahme und/oder Behandlung beschreibenden Vorgangsinformation ermittelt und in der Steuereinrichtung (11) hinterlegt wird.

6. Verfahren nach einem der vorangehenden Ansprüche, **dadurch gekennzeichnet, dass** mehrere Zielpositionen in der Steuereinrichtung (11) hinterlegt sind, welche während einer von der medizintechnischen Einrichtung durchzuführenden Bildaufnahme und/oder Behandlung zeitversetzt für eine mehrere automatische Positionierungen der Patientenlagerungseinrichtung umfassende Positionierungsfolge verwendet werden.

7. Verfahren nach einem der vorangehenden Ansprüche, **dadurch gekennzeichnet, dass** wenigstens ein der medizintechnischen Einrichtung (1) zugeordnetes Zusatzgerät (12), insbesondere ein Messaufnehmer, verwendet wird, wobei über ein Positionsbestimmungsmittel des Zusatzgerätes eine Bestimmung einer Zusatzgeräteposition erfolgt und die Zielposition durch die Steuereinrichtung (11) in Abhängigkeit von der Zusatzgeräteposition ermittelt wird.

8. Verfahren nach Anspruch 7, **dadurch gekennzeichnet, dass** als Positionsbestimmungsmittel ein optisches Positionsbestimmungsmittel des Zusatzgerätes (12), insbesondere wenigstens ein Lichtsender und/oder ein Lichtempfänger, verwendet wird, wobei die Zusatzgeräteposition durch die optische Positionsbestimmungseinrichtung der medizintechnischen Einrichtung (1) bestimmt wird.

9. Verfahren nach Anspruch 7 oder 8, **dadurch gekennzeichnet, dass** während der von der medizintechnischen Einrichtung (1) durchzuführenden Bildaufnahme und/oder Behandlung wenigstens eine aktualisierte Zielposition in Abhängigkeit von der Zusatzgeräteposition des wenigstens einen Zusatzgerätes (12) ermittelt und in der Steuereinrichtung (11) hinterlegt wird, wobei bei einer Positionsabweichung zwischen der Momentanposition des Patienten (2) und der wenigstens einen aktualisierten Zielposition eine erneute automatische Positionierung der Patientenlagerungseinrichtung erfolgt.

10. Verfahren nach einem der vorangehenden Ansprüche, **dadurch gekennzeichnet, dass** bei jeder automatischen Positionierung der Patientenlagerungseinrichtung eine diese Positionierung und/oder die Momentanposition des Patienten (2) und/oder die Zielposition beschreibende Positionierungsinformation in der Steuereinrichtung (11) gespeichert wird.

11. Medizintechnische Einrichtung (1) eingerichtet zur Durchführung eines Verfahrens nach einem der Ansprüche 1 bis 10, wobei die medizintechnische Einrichtung wenigstens eine eine dreidimensionale Lichtvorhanganordnung umfassende optische Positionsbestimmungseinrichtung, eine Steuereinrichtung (11) und eine Patientenlagerungseinrichtung umfasst, wobei die dreidimensionale Lichtvorhanganordnung mehrere Lichtvorhänge (4, 5) umfasst, deren Erfassungsbereiche sich zumindest teilweise unter einem Winkel schneiden, wobei ein erster Lichtvorhang (4) einen im Wesentlichen parallel zu einer zur Patientenlagerung vorgesehenen Fläche der Patientenlagerungseinrichtung angeordneten Erfassungsbereich (7)aufweist und ein zweiter Lichtvorhang (5) einen unter einem Winkel zu der zur Patientenlagerung vorgesehenen Fläche der Patientenlagerungseinrichtung angeordnetem Erfassungsbereich (9) aufweist.

12. Computerprogramm umfassend Befehle, welche bei Ausführung des Programms durch eine Steuereinrichtung (11), welche entweder Teil der medizintechnischen Einrichtung (1) gemäß Anspruch 11 oder mit dieser verbunden ist, diese veranlassen, ein Verfahren nach einem der Ansprüche 1 bis 10 auszuführen.

13. Computerlesbares Speichermedium, welches ein Computerprogramm nach Anspruch 12 enthält.

## Claims

1. Method for automatic positioning of a patient (2) relative to a medical device (1), wherein the patient (2) is positioned on a patient setup device of the medical device (1) in a detection area of an optical position determining device of the medical device (1), said optical position determining device comprising at least one three-dimensional light curtain arrangement, wherein a start position of the patient (2) is detected by the optical position determining device and a position deviation between the start position detected and a target position stored in a control device (11) is determined, wherein, depending on the position deviation, the patient setup device is automatically positioned in the target position in up to three dimensions, **characterised in that** a three-dimensional light curtain arrangement is used which comprises a plurality of light curtains (4, 5), the detection areas of which at least partially intersect at an angle, wherein a first light curtain (4) having a detection area (7) disposed essentially parallel to a patient setup device surface provided for patient positioning and a second light curtain (5) having a detection area (9) disposed at an angle to the patient setup device surface provided for patient positioning are used.

2. Method according to claim 1, **characterised in that** the second light curtain (5) at least partially penetrates a patient aperture (10) of the medical device (1).

3. Method according to claim 1 or 2, **characterised in that**, after the initial automatic positioning of the patient setup device, an instantaneous position of the patient (2) is detected by the optical position determining device during imaging and/or treatment to be carried out by the medical device (1).

4. Method according to claim 3, **characterised in that**, if a position deviation between the instantaneous position of the patient (2) and the at least one target position stored in the control device (11) occurs during the imaging and/or treatment, the patient setup device is automatically re-positioned.

5. Method according to one of the preceding claims, **characterised in that** the target position is determined by the control device (11) in accordance with process information describing imaging and/or treatment to be carried out by the medical device (1) and is stored in the control device (11).

6. Method according to one of the preceding claims, **characterised in that** a plurality of target positions are stored in the control device (11) which are used at different times during imaging and/or treatment to be carried out by the medical device for a positioning sequence comprising a plurality of automatic positionings of the patient setup device.

7. Method according to one of the preceding claims, **characterised in that** at least one accessory unit (12) assigned to the medical device (1), in particular a sensor, is used, wherein an accessory unit position is determined via position determining means of the accessory unit and the target position is determined by the control device (11) according to the accessory unit position.

8. Method according to claim 7, **characterised in that** an optical position determining means of the accessory unit (12), in particular at least one light transmitter and/or light receiver, is used as position determining means, wherein the accessory unit position is determined by the optical position determining device of the medical device (1).

9. Method according to claim 7 or 8, **characterised in that** during the imaging and/or treatment to be carried out by the medical device (1) at least one updated target position is determined according to the accessory unit position of the at least one accessory unit (12) and stored in the control device (11), wherein, in the event of a position deviation between the instantaneous position of the patient (2) and the at least one updated target position, the patient setup device is automatically re-positioned.

10. Method according to one of the preceding claims, **characterised in that** for each automatic positioning of the patient setup device, positioning information describing this positioning and/or the instantaneous position of the patient (2) and/or the target position is stored in the control device (11) .

11. Medical device (1) designed to carry out a method according to one of claims 1 to 10, wherein the medical device comprises at least one optical position determining device comprising a three-dimensional light curtain arrangement, a control device (11) and a patient setup device, wherein the three-dimensional light curtain arrangement comprises a number of light curtains (4, 5), the detection areas of which intersect at least partially at an angle, wherein a first light curtain (4) has a detection area (7) disposed essentially parallel to a patient setup device surface provided for patient positioning and a second light curtain (5) has a detection area (9) disposed at an angle to the patient setup device surface provided for patient positioning.

12. Computer program comprising instructions which, when the program is executed by a control device (11), which is either part of the medical device (1) according to claim 11 or is connected hereto, cause it to carry out a method according to one of claims 1 to 10.

13. Computer-readable storage medium which contains a computer program according to claim 12.

## Revendications

1. Procédé de mise en position automatique d'un patient (2) par rapport à un dispositif (1) de la technique médicale, le patient (2) étant dans une région de détection d'un dispositif optique de définition de la position, comprenant au moins un système à rideaux lumineux tridimensionnel, du dispositif (1) de la technique médicale couché sur un dispositif de couchage de patient du dispositif (1) de la technique médical, dans lequel on détermine une position initiale du patient (2) par le dispositif optique de définition de la position et on détermine un écart de position entre la position initiale déterminée et une position visée mise en mémoire dans un dispositif (11) de commande, dans lequel une mise en position automatique du dispositif de couchage de patient dans la position visée s'effectue jusqu'à trois dimensions en fonction de l'écart de position, **caractérisé en ce que** l'on utilise un système à rideaux lumineux tridimensionnel, qui comprend plusieurs rideaux (4, 5) lumineux, dont les régions de détection se coupent, au moins en partie, suivant un angle, dans lequel on utilise un premier rideau (4) lumineux ayant une région (7) de détection sensiblement parallèle à une surface prévue pour le couchage du patient du dispositif de couchage du patient, ainsi qu'un deuxième rideau (5) lumineux ayant une région (9) de détection faisant un angle avec la surface prévue pour le couchage du patient du dispositif de couchage du patient.

2. Procédé suivant la revendication 1, **caractérisé en ce que** le deuxième rideau (5) lumineux passe, au moins en partie, dans un logement (10) de patient du dispositif (1) de la technique médicale.

3. Procédé suivant la revendication 1 ou 2, **caractérisé en ce que**, pendant une prise de vue effectuée par le dispositif (1) de la technique médicale et/ou un traitement après la première mise en position automatique du dispositif de couchage du patient, on détermine, en continu, une position instantanée du patient (2) par le dispositif optique de définition de la position.

4. Procédé suivant la revendication 3, **caractérisé en ce que**, s'il y a un écart de position se produisant pendant la prise de vue et/ou le traitement entre la position instantanée du patient (2) et la au moins une position visée mise en mémoire dans le dispositif (11) de commande, il s'effectue une mise en position automatique renouvelée du dispositif de couchage du patient.

5. Procédé suivant l'une des revendications précédentes, **caractérisé en ce que** l'on détermine, par le dispositif (11) de commande, la position visée, en fonction d'une information opératoire décrivant la prise de vue et/ou le traitement effectué par le dispositif (1) de la technique médicale, et on la met en mémoire dans le dispositif (11) de commande.

6. Procédé suivant l'une des revendications précédentes, **caractérisé en ce que** l'on met en mémoire, dans le dispositif (11) de commande, plusieurs positions visées, qui, pendant une prise de vue d'images et/ou une manipulation effectuée par le dispositif de la technique médicale, sont utilisées de manière décalée dans le temps pour une succession de mises en position comprenant plusieurs mises en position automatiques du dispositif de couchage du patient.

7. Procédé suivant l'une des revendications précédentes, **caractérisé en ce que** l'on utilise au moins un appareil (12) supplémentaire, notamment un capteur de mesure, associé au dispositif (1) de la technique médicale, une définition d'une position d'appareil supplémentaire s'effectuant par un moyen de définition de la position de l'appareil supplémentaire, et l'on détermine, par le dispositif (11) de commande, la position visée en fonction de la position de l'appareil supplémentaire.

8. Procédé suivant la revendication 7, **caractérisé en ce que** l'on utilise, comme moyen de définition de la position, un moyen optique de définition de la position de l'appareil (12) supplémentaire, notamment au moins un émetteur de lumière et/ou un récepteur de lumière, la position de l'appareil supplémentaire étant déterminée par le dispositif optique de définition de la position du dispositif (1) de la technique médicale.

9. Procédé suivant la revendication 7 ou 8, **caractérisé en ce que**, pendant la prise de vue et/ou le traitement effectué par le dispositif (1) de la technique médicale, on détermine une position visée mise à jour, en fonction de la position du au moins un appareil (12) supplémentaire, et on la met en mémoire dans le dispositif (11) de commande, dans lequel, s'il y a un écart de position entre la position instantanée du patient (2) et la au moins une position visée mise à jour, il s'effectue une mise en position automatique renouvelée du dispositif de couchage du patient.

10. Procédé suivant l'une des revendications précédentes, **caractérisé en ce que**, à chaque mise en position automatique du dispositif de couchage du patient, on met en mémoire, dans le dispositif (11) de commande, une information de mise en position décrivant cette mise en position et/ou la position instantanée du patient (2) et/ou la position visée.

11. Dispositif (1) de la technique médicale conçu pour effectuer un procédé suivant l'une des revendications 1 à 10, le dispositif de la technique médicale comprenant, au moins, un dispositif optique de définition de la position, comprenant un système à rideaux lumineux tridimensionnel, un dispositif (11) de commande et un dispositif de couchage du patient, le système à rideaux lumineux tridimensionnel comprenant plusieurs rideaux (4, 5) lumineux, dont les régions de détection se coupent, au moins en partie, suivant un angle, dans lequel un premier rideau (4) lumineux a une région (7) de détection, sensiblement parallèle à une surface prévue pour le couchage du patient du dispositif de couchage du patient, et un deuxième rideau (5) lumineux a une région (9) de détection faisant un angle avec la surface prévue pour le couchage du patient du dispositif de couchage du patient.

12. Programme d'ordinateur comprenant des instructions, qui, lors de la réalisation du programme par un dispositif (11) de commande, qui soit fait partie du dispositif (1) de la technique médicale suivant la revendication 11, soit y est relié, fait que celui-ci exécute un procédé suivant l'une des revendications 1 à 10.

13. Support de mémoire déchiffrable par ordinateur, qui contient un programme d'ordinateur suivant la revendication 12.
